(19) Europäisches Patentamt
European Patent Office
Office européen des brevets

(11) Publication number: **0 050 916**
**A2**

# (12) EUROPEAN PATENT APPLICATION

(21) Application number: 81304143.1

(22) Date of filing: 10.09.81

(51) Int. Cl.³: C 07 D 271/04
C 07 D 295/12, C 07 C 121/43
A 61 K 31/41, A 61 K 31/445
A 61 K 31/495, A 61 K 31/535
A 61 K 31/40, A 61 K 31/275

(30) Priority: 02.10.80 US 193043
18.05.81 US 264892

(43) Date of publication of application: 05.05.82 Bulletin 82/18

(84) Designated Contracting States: AT BE CH DE FR IT LI LU NL SE

(71) Applicant: AMERICAN HOME PRODUCTS CORPORATION
685, Third Avenue
New York, New York 10017(US)

(72) Inventor: Stein, Reinhardt Peter
2761, Lantern Lane
Audubon Pennsylvania 19407(US)

(74) Representative: Wileman, David Francis et al,
c/o John Wyeth and Brother Limited Huntercombe Lane South
Taplow Maidenhead Berkshire SL6 OPH(GB)

## (54) CNS stimulants and antihypertensive agents.

(57) Compounds of formula

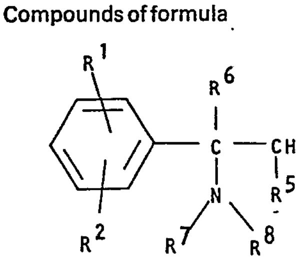

(1a)

in which R represents one of the following:

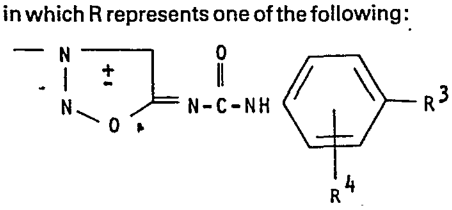

,

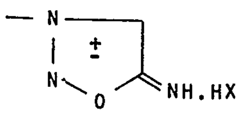

or $-N(NO)-CH_2CN$

wherein X is the anion of a strong acid having a pKa below 2; $R^1$ and $R^2$ are, independently, hydrogen, alkyl of 1 to 6 carbon atoms, alkoxy of 1 to 6 carbon atoms, halo, perfluoroalkyl of 1 to 3 carbon atoms, nitro, alkanoyl of 2 to 4 carbon atoms or alkoxycarbonyl of 2 to 4 carbon atoms; $R^3$ is hydrogen, halo, nitro or alkanoyl of 2 to 4 carbon atoms; $R^4$ is hydrogen, halo, nitro or perfluoroalkyl of 1 to 3 carbon atoms; $R^5$ and $R^6$ are, independently, hydrogen or methyl; and $R^7$ and $R^8$ are, independently, alkyl of 1 to 4 carbon atoms, or when taken with the nitrogen atom to which they are attached form a piperidinyl, pyrolidinyl, morpholinyl, *N*-alkyl piperazinyl in which the alkyl group contains from 1 to 6 carbon atoms or *N*-phenylpiperazinyl group; or a pharmaceutically acceptable acid addition salt thereof are disclosed which have CNS stimulant and antihypertensive activity. Processes for their preparation and pharmaceutical compositions are also described.

EP 0 050 916 A2

This invention relates to compounds which are CNS stimulants and antihypertensive agents, to processes for their preparation and to pharmaceutical compositions containing them.

After the discovery of the central nervous system stimulatory properties of 3-(1-methyl-2-phenylethyl)-N-(phenylcarbamoyl)sydnone imine (Sydnocarb; U.S.S.R. 329,890 and Offenlegungsschrift 2,028,880) various analogues have been reported. U.S.S.R. 222,370 and Offenlegungsschrift 2,738,022 disclose sydnone imines which contain phenyl, 1- or 2-phenylethyl and the phenylisopropyl groups in 3-position as well as N-meta- and para-chlorophenyl and N-phenyl carbamoyl groups. Variations of 3-benzyl sydnonimines are disclosed in U.S. 3,277,108. Other variously substituted 3-aralkyl sydnonimines are disclosed by Olovyanishinkiva et al., Khim. Geterotsikl Soedin, 2 170-175 (1978) and 9 1198-1203 (1975).

Sydnocarb is conventionally produced by cyanomethylation of amphetamine followed by nitrosation and ring closure with a mineral acid yielding sydnophen as an acid halide salt which is reacted with phenylisocyanate under mildly basic conditions to introduce the N-phenylcarbamoyl group. As an asymmetric compound, amphetamine may be employed as the initial reactant as the racemic d,l-mixture or as the pure d- or l-isomer to yield racemic or optically active sydnophen and ultimately sydnocarb.

Yashunskii et al., J. Med. Chem., 14 1013-1015 (1971) disclose the marked CNS-stimulatory effect of 3-(1-methyl-2-phenylethyl) sydnonimine (Sydnophen). The relative activities of a large number of alkyl, aryl and aralkylsydnonimines are presented in Table 1 on page 1014. Most of them, including compound XVIII (2-hydroxy-1-methyl-2-phenylethyl-sydnonimine), were essentially inactive central nervous system stimulants relative to compound XIII (Sydnophen), demonstrating the criticality of the structure of the 3-substituent in

the Sydnocarb series of compounds as far as CNS stimulatory activity is concerned. Thus, although the activity profile of Sydnocarb is not identical to that of amphetamine, or for that matter Sydnophen, CNS stimulatory activity is a common property of the initial reactant amphetamine, the intermediate Sydnophen and the final product Sydnocarb.

Although Sydnocarb and its derivatives disclosed in the literature forms salts with various organic and inorganic acids, such salts are not appreciably water soluble and when stirred in water, the complex or adduct salt is broken up to reisolate the neutral mesoionic sydnone imine substrate.

The metabolites of Sydnocarb have been studied by several groups. L.E. Kholodov and E.T. Lilin, Mater. Resp. Rasshir. Konf. Farmacol. Gruz. 2nd 1977, 84-5 report finding hydroxylation of Sydnocarb at the beta carbon of the phenylisopropyl substituent and at the phenyl ring of the phenylcarbamoyl group, hydrolytic cleavage of the phenylcarbamoyl group and ring opening of the heterocyclic nucleus. They report that the psychostimulating activity of Sydnocarb is a property of that compound and not its metabolites. Polgar et al. Acta. Pharm. Hung., 48, Suppl. 23-24 (1978) and Xenobiotica 9, No. 8, 511-520 (1979) report several hydroxylated metabolites and conjugates of hydroxylated Sydnocarb.

It has been suggested by Kikuchi et al. Jap. J. Pharmac. 20 23-43 (1970) that sydnonimines containing an amine in 3-position produce hypotension while sydnonimines containing an alkyl, cycloalkyl or dialkylaminoalkyl group in 3-position produce hypertension.

This invention provides compoundsof formula:

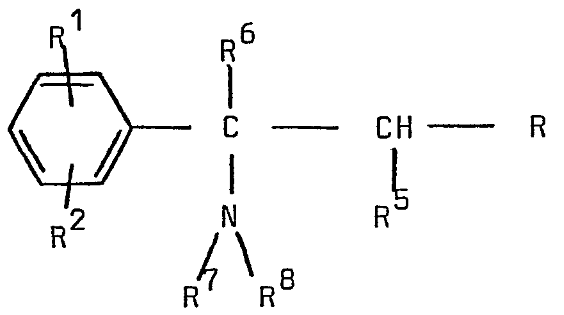

(Ia)

in which R represents one of the following:

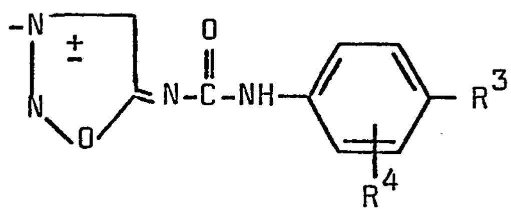

;

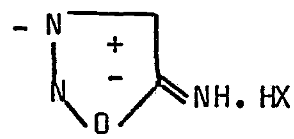

or $-N(NO)-CH_2CN$

wherein X is the anion of a strong acid having a pKa below 2; $R^1$ and $R^2$ are, independently, hydrogen, alkyl of 1 to 6 carbon atoms, alkoxy of 1 to 6 carbon atoms, halo, perfluoroalkyl of 1 to 3 carbon atoms, nitro, alkanoyl of 2 to 4 carbon atoms or alkoxycarbonyl of 2 to 4 carbon atoms; $R^3$ is hydrogen, halo, nitro or alkanoyl of 2 to 4 carbon atoms; $R^4$ is hydrogen, halo, nitro or perfluoroalkyl of 1 to 3 carbon atoms; $R^5$ and $R^6$ are, independently, hydrogen or methyl; and $R^7$ and $R^8$ are, independently, alkyl of 1 to 4 carbon atoms, or when taken with the nitrogen atom to which they are attached form a piperidinyl, pyrolidinyl, morpholinyl, N-alkyl piperazinyl in which the alkyl group contains from 1 to 6 carbon atoms or N-phenylpiperazinyl group; or a pharmaceutically acceptable acid addition salt thereof.

In accordance with this invention there is provided a group of central nervous system (CNS) stimulants which are 3-(2-amino-2-phenylethyl)-N-[(phenylamino)carbonyl]-sydnonimines optionally substituted in either or both phenyl rings, of the formula:

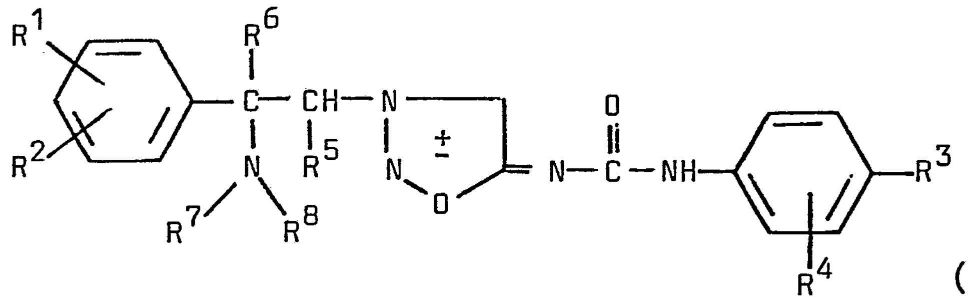

(I)

in which $R^1$, $R^2$, $R^3$, $R^4$, $R^5$, $R^6$, $R^7$ and $R^8$ are as defined above, or a pharmaceutically acceptable acid addition salt thereof.

In addition, there is provided a group of 5-amino-3-[2-(substituted amino)-2-phenylethyl]-1,2,3-oxadiazolium salts and [[2-(substituted amino)-2-phenylethyl]-nitrosoamino]-acetonitrile derivatives which are intermediates useful in the production of the CNS stimulants disclosed in the preceding paragraph, which intermediates are, in their own right, antihypertensive agents useful in the treatment of hypertension.

The oxadiazolium salts of this invention are represented by the following structural formula:

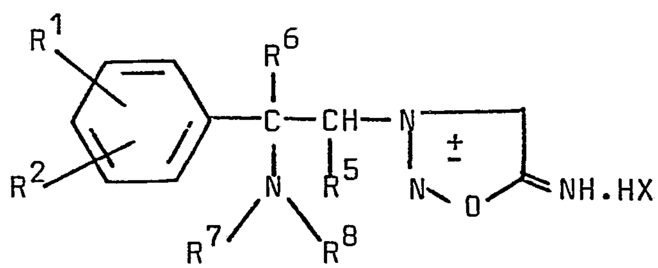

(II)

in which $R^1$, $R^2$, $R^5$, $R^6$, $R^7$ and $R^8$ are as defined above and X is the anion of a strong acid having a pKa below 2.

The oxadiazolium salts of this invention revert to the corresponding nitroso-nitrile precursor via a pH dependent ring opening. Ring opening occurs rapidly under basic conditions and slower in mild acid, in essence demonstrating the reverse of the ring closing accomplished with strong acids. The nitroso-nitriles are, both intermediates for the antihypertensive oxadiazolium salts and active antihypertensive agents in their own right.

Thus, in accordance with this invention there is also provided a group of nitroso-nitriles useful as antihypertensive agents and in the production oxadiazolium antihypertensive salts, of the formula:

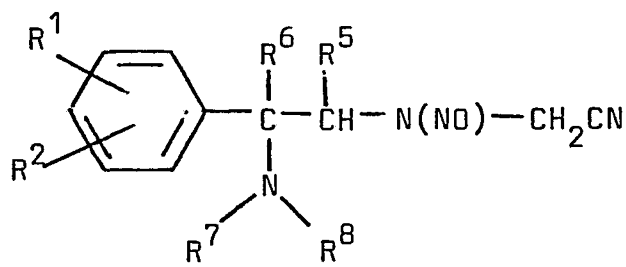

(III)

in which $R^1$, $R^2$, $R^5$, $R^6$, $R^7$ and $R^8$ are as defined above, or a pharmaceutically acceptable salt thereof.

In the preceding structural formulae, it is generally preferred that the halo substituent be chlorine, bromine or fluorine although iodine is acceptable. Likewise, it is preferred that the alkyl and alkoxy substituents be relatively small, the methyl, ethyl, propyl, isopropyl, methoxy, ethoxy, propoxy and isopropoxy groups being preferred.

The pharmaceutically acceptable acid addition salts of the compounds of formulae II and III are conventionally produced by the method and from any of the acids disclosed in U.S. 3,277,108, which exhibit a pKa below 2. The salt is preferably that formed during cyclization of the nitrosonitrile, such as with hydrochloric, hydrobromic, sulfuric, phosphoric, methane sulfonic, p-toluene sulfonic acid, and the like. The anion can subsequently be replaced with a desired anion of ion exchange chromatography following conventional procedures. The salts of the compounds of formulae I may be produced with milder acids such as acetic, propionic, oxalic, succinic, maleic, fumeric acid, and the like, as well as with the strong acids previously mentioned in connection with the compounds of formulae II and III. The salts formed with the aminic bases of this invention are generally water soluble, dissociating sufficiently to dissolve in aqueous medium to provide a homogeneous solution. Thus, the compounds of this invention may be formulated for administration in aqueous vehicle for practical dosing to reduce blood pressure in patients unable to receive treatment orally.

The compounds of this invention contain one chiral center when $R^5$ is hydrogen and two chiral centers when $R^5$ is methyl. Thus, depending upon the identity of the substituent $R^5$, there is obtained either one or two racemic mixtures of product. The epimers and optical isomers are readily separable by standard techniques well known to the chemist. By selection of the desired starting material, the product can be limited to a single racemic mixture of isomers.

The oxadiazolium salts and the nitroso-nitriles of this invention are prepared by conventional techniques employed in the preparation of sydnonimines.

The starting materials are either known or preparable by routine synthetic methods. Thus, a properly substituted 2-tertiary amino-2-phenylethyl amine is

cyanomethylated with a reactant $XCH_2CN$ where X may be -OH, -Br, -Cl, tosyl, and the like to form the intermediate-

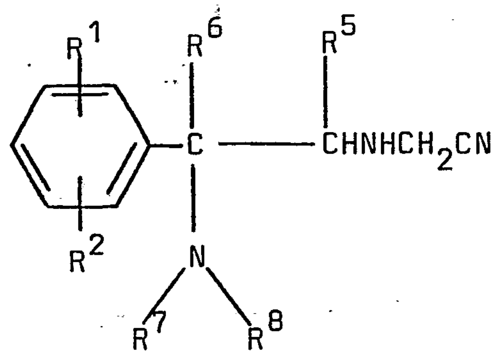

(V)

which is nitrosated, e.g. with an excess of $NaNO_2$ in aqueous HCl, to yield the nitroso-nitrile-

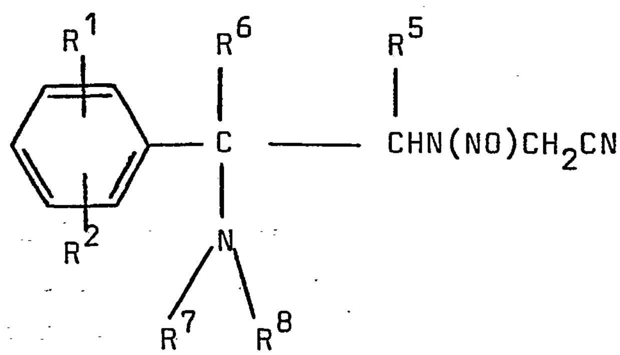

(III)

in which formulae $R^1$, $R^2$, $R^5$, $R^6$, $R^7$ and $R^8$ are as defined above. The compound of formula III may be reacted with excess strong acid to form the oxadiazolium salt-

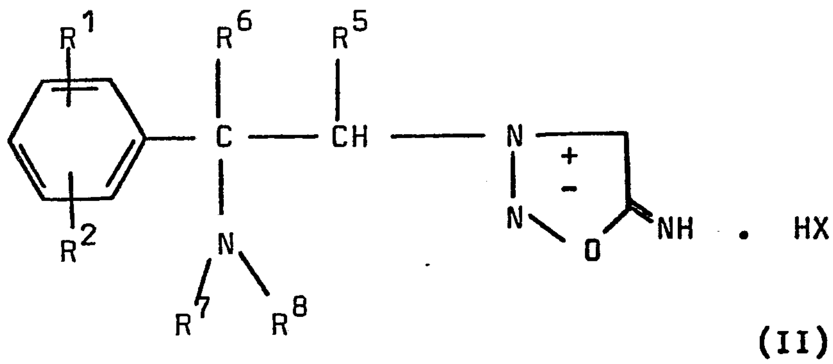

(II)

wherein X, $R^1$, $R^2$, $R^5$, $R^6$, $R^7$ and $R^8$ are as hereinbefore defined. The compounds of formula I are produced from either the nitroso-nitrile of formula III or the oxadiazolium salt of formula II by reaction with an isocyanate-

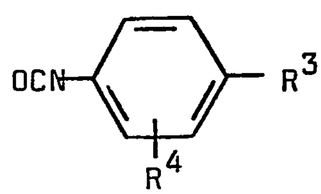

in which formulae $R^{1-8}$ and X are as hereinbefore defined, in the presence of an organic amine base such as triethylamine, 4-dimethylaminopyridine, and the like, following the procedure disclosed in Offenlegungsschrift 2,738,022. In theory, the base selected must be sufficiently alkaline to neutralize the oxadiazolium salt (including the aminic salt) which permits ring opening and reformation of the nitroso-nitrile which in turn undergoes nucleophilic addition of the isocyanate.

This invention also provides a pharmaceutical composition comprising a compound of formula Ia or a pharmaceutically acceptable salt thereof and a pharmaceutically acceptable carrier. Any suitable carrier known in the art may be employed in such

compositions.

The following Example illustrates the preparation of d,l-[[2-(dimethylamino)-2-phenylethyl]nitrosoamino]-acetonitrile and its use as an intermediate in the production of d,l-5-amino-3-[2-(dimethylamino)-2-phenylethyl]-1,2,3-oxadiazolium chloride.

## Example 1

### dl-5-Amino-3-[2-(dimethylamino)-2-phenylethyl]-1,2,3-oxadiazolium chloride

a) Dissolve dl-2-dimethylamino-2-phenylamine, dihydrochloride (4.74 g.) in water (50 ml.), stir and cool with an ice-bath. Add 37% aqueous formaldehyde solution (2.0 ml.), stir for 10 minutes, then drip in a solution of potassium cyanide (1.30 g.) in water (20 ml.). Stir the cold solution for 1 hour, then cool further to $0^{o}$ C. (ice-salt bath). Drip in a solution of sodium nitrite (1.4 g.) in water (15 ml.) followed by 5N aqueous HCl (8 ml.). Stir, then again drip in a solution of sodium nitrite (1.4 g.) in water (15 ml.) and continue stirring for 3 hours, allowing the reaction to warm to room temperature. Drip in 5N aqueous

sodium hydroxide solution until a pH of 10 is attained. Quickly extract with diethyl ether, then dry and evaporate the solvent *in vacuo*. Pump dry, then treat the oil in diethyl ether with decolorizing carbon, filter and evaporate, then pump to obtain *dl*-[[2-(dimethylamino)-2-phenylethyl]nitrosoamino]acetonitrile as an oil (about 3 g.). To characterize this product dissolve a sample (313 mg.) in methylene chloride, treat with 5N isopropanolic-HCl (1 ml.), then evaporate the solvent *in vacuo*. Crystallize the residue from acetone, filter to obtain the hydrochloride salt (210 mg.); m.p. 164-166° C.

Analysis for: $C_{12}H_{16}N_4O \cdot HCl$
Calculated: C, 53.63; H, 6.38; N, 20.85%
Found: C, 53.51; H, 6.34; N, 21.31%

b) Dissolve dl-[[2-(dimethylamino)-2-phenylethyl]nitrosoamino]acetonitrile (4.43 g.) in methylene chloride, add excess 5N isopropanolic - HCl (8 ml.) and let stand overnight. Filter to obtain 1.86 g. of the crude title product, m.p. 150° C (dec).

Combine the product of several runs (3.34 g.), dissolve in 70% aqueous ethanol, treat with decolorizing carbon, filter and dilute the filtrate with isopropanol. Evaporate the solvents *in vacuo*, cover the remaining glassy material with methylene chloride and let stand to crystallize. Filter to obtain 2.62 g. of the title product as the hydrochloride hydrate, m.p. 157-158° C (dec).

Analysis for: $C_{12}H_{17}ClN_4O \cdot HCl\ 1.5H_2O$
Calculated: C, 43,38; H, 6.37; N, 16.86%
Found: C, 43.66; H, 5.85; N, 17.49%

c) The antihypertensive activity of *d,l*-5-amino-3-(2-(dimethylamino)-2-phenylethyl)-1,2,3-oxadiazolium chloride, hydrochloride, which compound is representative in its activity of the compounds of formulae II and III, was established by orally administering the compound to a group of unanesthetized, spontaneously

hypertensive rats while indirectly measuring their systolic blood pressure employing a Decker Caudal Plethysmograph. A decrease of 51 mmHg blood pressure was found at 1.5 hours after oral administration of 50 mg/kg of compound and a decrease of 40 mmHg at 1.5 and 4 hours at 25 mg/kg was demonstrated.

Thus, the antihypertensive agents of formulae II and III are useful in treatment of hypertension and as such they may be administered to a patient suffering from hypertension, orally or parenterally, in an amount of from about 25 to 50 mg/kg or more, based upon the test results, in single or divided doses. The dosage regimen and route of administration may be varied by the attending physician to achieve the desired response depending upon the condition of the patient relative to age, severity of hypertensive state, etc.

The following Examples illustrate, without limitation, the process for directly producing the compounds of formula I. Where the intermediate cyanomethylated product of the phenethyl amine and the N-nitroso derivative thereof are isolated as oils, no attempt was made to obtain the purified intermediate. The activity counts presented at the end of each example represent the difference from control based upon the test procedure disclosed, infra. dl-Sydnocarb demonstrated a difference from control of 939 activity counts at 10 mg/kg., p.o.

Example 2

dl-3-[2-(Dimethylamino)-2-phenylethyl]-N-[(phenylamino)-carbonyl]sydnone imine

Stir the crude oily free base, dl-N-nitroso-N-[2-(dimethylamino)-2-phenylethyl]amino acetonitrile (10.2 g.) prepared according to Example 1 (a) with toluene (150 ml.), add phenylisocyanate (5.75 g.) followed by triethylamine (4.44 g.) and heat the mixture at 55°C for 4 hours. Cool and let stand at room temperature. Evaporate the solvent *in vacuo* and pump to obtain an oil. Treat the oil in ethyl acetate with decolourising carbon, filter, then treat the filtrate with 5N isopropanolic-HCl (20 ml.). Evaporate the solvent *in vacuo* without the application of heat, triturate the gum with isopropanol containing a little methylene chloride to initiate crystallisation. Let stand, then filter to obtain 16.7 g. of the crude title product; m.p. 139-143° C. (dec). Dissolve the solid in methylene chloride containing a little methanol, treat with decolourising carbon, filter and evaporate the solvents *in vacuo*. Cover the oil with acetonitrile, then scratch and triturate to fully crystallize and filter to obtain 8.0 g. of the pure title product as a dihydrochloride salt partial hydrate; m.p. 149-151° C. (dec).

| | |
|---|---|
| Analysis for: | $C_{19}H_{21}N_5O_2.2HCl.1/3H_2O$ |
| Calculated: | C, 53.03; H, 5.54; N, 16.28% |
| Found: | C, 53.17; H, 5.42; N, 16.41% |
| Activity Counts: | 859 at 10 mg/kg. |

Example 3

dl-3-(2-Dimethylamino-2-phenylethyl)-N-[4-chlorophenylamino)carbonyl]sydnone imine,

The title compound, dihydrochloride salt, is prepared following the procedure of Example 2 with the exception that p-chlorophenylisocyanate is employed as the reactant rather

than phenylisocyanate, m.p. 150-153° C.
Analysis for: $C_{19}H_{20}N_5O_2Cl \cdot 2HCl \cdot 1.5H_2O$
Calculated: C, 46.97; H, 5.19; N, 14.42
Found: C, 46.62; H, 4.86; N, 14.18
Activity Counts: 1329 at 10 mg/kg and 431 at 1 mg/kg.

The activity profile of the compounds of formula I is similar to that of amphetamine in some aspects while being devoid of other activities of amphetamine. For example, like amphetamine the compounds of this invention increase motor activity. However, the compounds of this invention are much less toxic than amphetamine providing a slower onset of activity (which indicates less euphoria and abuse potential).

The compounds of formula I were shown to possess central nervous system stimulant activity by subjecting them to the following standard test procedure:

Male mice weighing 17 to 25 gms. are injected orally with drug solubilised or suspended in 1% Tween 80. Control animals are injected with 1% Tween 80.

Six Columbus Instrument Company activity chambers are employed. Three mice given identical treatment are placed in each chamber for all tests. During each run, control animals (1% Tween only) occupy 3 chambers; the other 3 chambers measure activity of drug treated animals. For each dose of a given drug the experiment is run two times in a counterbalanced design so that each specific activity chamber records the activity of control animals during one run, and the activity of drug animals on the other run. Thus at each dose level 18 mice are used in the drug group and 18 mice in the control group.

Activity counts are recorded every ten minutes for a period of 2 hours. The data are analysed using Students "t" test comparing the means of the control and drug groups for each 10 minute period. The drug treated group is compared graphically with the control group in regard to duration of action and dose response at peak drug activity.

As central nervous system stimulants with unique activity profiles, the compounds of formula I are useful in the treatment of anergic disorders (such as sleepiness and fatigue) including related types of depression and narcolepsy. Based upon the potency of the compounds of formula I in use in mice, the dose contemplated for use in the 70 kilogram human would vary from about 35-700 milligrams administered orally once or twice per day under the guidance of a physician. Of course, the dosage regimen as well as the route of administration, oral or parenteral, will vary with the condition of the patient relative to age, severity of depression, etc.

CLAIMS

1. A compound of formula

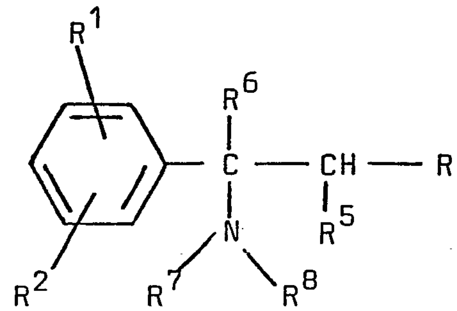

(Ia)

in which R represents one of the following:

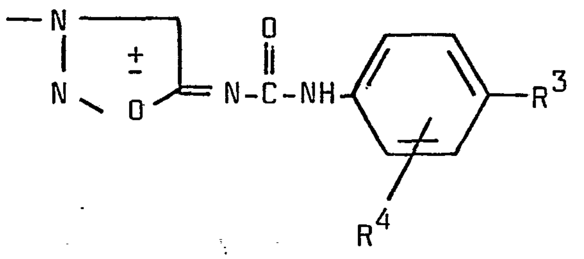

,

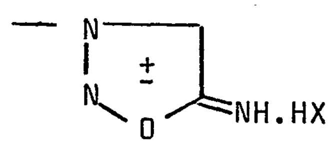

or $-N(NO)-CH_2CN$

wherein X is the anion of a strong acid having a pKa below 2; $R^1$ and $R^2$ are, independently, hydrogen, alkyl of 1 to 6 carbon atoms, alkoxy of 1 to 6 carbon atoms, halo, perfluoroalkyl of 1 to 3 carbon atoms, nitro, alkanoyl of 2 to 4 carbon atoms or alkoxycarbonyl of 2 to 4 carbon atoms; $R^3$ is hydrogen, halo, nitro or alkanoyl of 2 to 4 carbon atoms; $R^4$ is hydrogen, halo, nitro or perfluoroalkyl of 1 to 3 carbon atoms; $R^5$ and $R^6$ are, independently, hydrogen or methyl; and $R^7$ and $R^8$ are, independently, alkyl of 1 to 4 carbon atoms, or when taken with the nitrogen atom to which they are attached form a piperidinyl, pyrrolidinyl, morpholinyl, N-alkyl

piperazinyl in which the alkyl group contains from 1 to 6 carbon atoms or N-phenylpiperazinyl group; or a pharmaceutically acceptable acid addition salt thereof.

2. A compound as claimed in Claim 1 wherein R represents

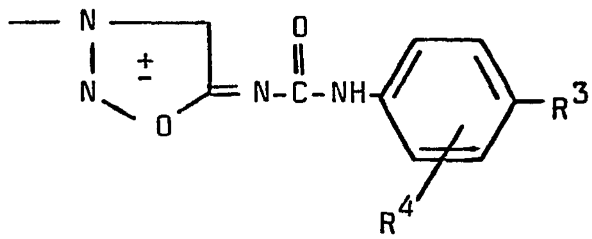

and $R^3$ is hydrogen or halogen.

3. A compound as claimed in Claim 1 or Claim 2 wherein $R^7$ and/or $R^8$ is methyl.

4. d-, l- or dl-3-(2-Dimethylamino-2-phenylethyl)-N-[(phenylamino)carbonyl]sydnone imine or a non-toxic acid addition salt thereof.

5. d-, l- or dl-3-(2-Dimethylamino-2-phenylethyl)-N-[(4-chlorophenylamino)carbonyl]sydnone imine or a non-toxic acid addition salt thereof.

6. 5-Amino-3-[2-(dimethylamino)-2-phenylethyl]-1,2,3-oxadiazolium chloride or a pharmaceutically acceptable acid addition salt thereof.

7. d,l-[[2-(Dimethylamino)-2-phenylethyl]nitrosoamino]-acetonitrile or a pharmaceutically acceptable acid addition salt thereof.

BAD ORIGINAL

8. A process for preparing a compound of formula Ia as defined in Claim 1 wherein R has the formula

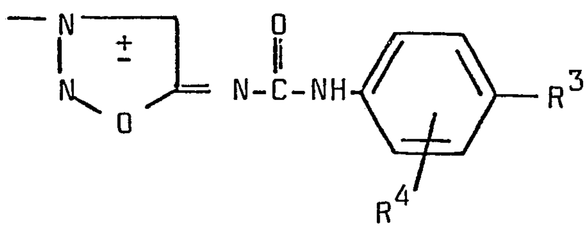

or a pharmaceutically acceptable salt thereof which comprises reacting an oxadiazolium salt of the formula:

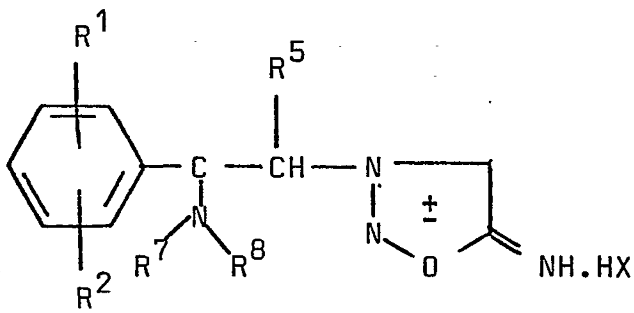

(II)

or the ring opened form of formula:

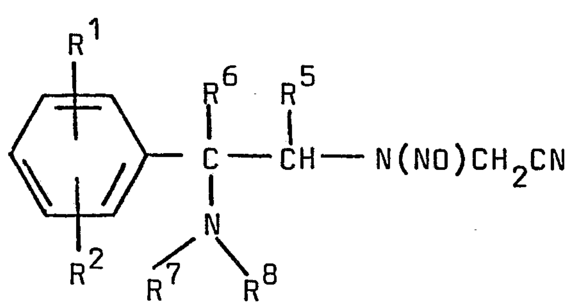

(III)

with an isocyanate of the formula:

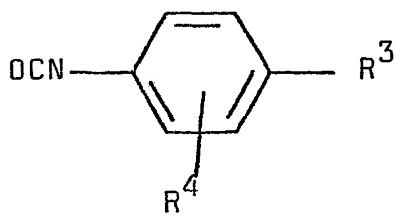

(IV)

in the presence of an organic amine base, in which formula X, $R^1$, $R^2$, $R^3$, $R^4$, $R^5$, $R^6$, $R^7$ and $R^8$ are as defined in Claim 1, if desired converting to a pharmaceutically acceptable acid addition salt.

9. A process for preparing a compound of formula Ia as claimed in Claim 1 wherein R has the formula

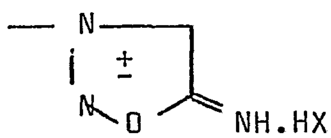

which comprises treating a nitrosonitrile of the formula:

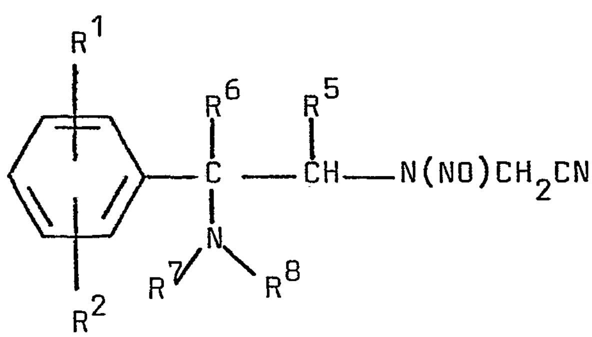

(III)

wherein $R^1$, $R^2$, $R^5$, $R^6$, $R^7$ and $R^8$ are as defined in Claim 1 with excess strong acid.

10. A process for preparing a nitroso-nitrile as claimed in Claim 1 wherein R has the formula $-N(NO)CH_2CN$ which comprises nitrosating a compound of the formula:

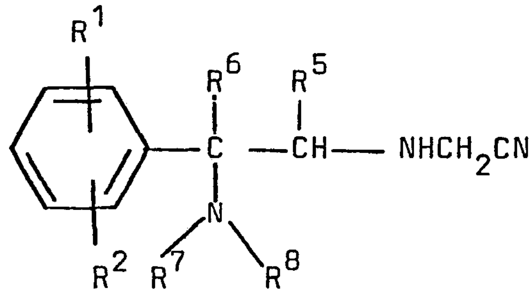

(V)

wherein $R^1$, $R^2$, $R^5$, $R^6$, $R^7$ and $R^8$ are defined in Claim 1 and if desired converting to a pharmaceutically acceptable salt.

11. A compound of formula Ia as claimed in Claim 1 for use as a CNS stimulant or antihypertensive agent.

12. A pharmaceutical composition comprising a compound of formula Ia as defined in Claim 1 or a pharmaceutically acceptable salt thereof and a pharmaceutically acceptable carrier.

CLAIMS FOR AUSTRIA

1. A process for preparing a compound of formula

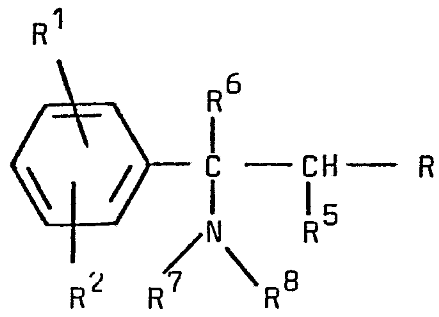

(Ia)

in which R represents one of the following:

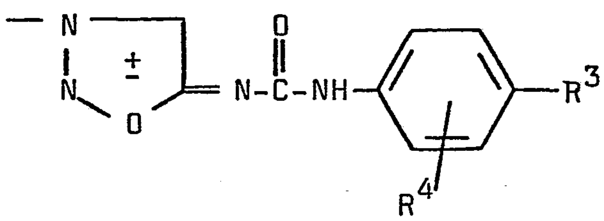

,

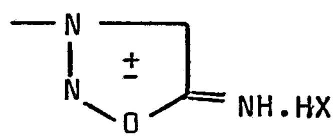

or $-N(NO)-CH_2CN$

wherein X is the anion of a strong acid having a pKa below 2; $R^1$ and $R^2$ are, independently, hydrogen, alkyl of 1 to 6 carbon atoms, alkoxy of 1 to 6 carbon atoms, halo, perfluoroalkyl of 1 to 3 carbon atoms, nitro, alkanoyl of 2 to 4 carbon atoms or alkoxycarbonyl of 2 to 4 carbon atoms; $R^3$ is hydrogen, halo, nitro or alkanoyl of 2 to 4 carbon atoms; $R^4$ is hydrogen, halo, nitro or perfluoroalkyl of 1 to 3 carbon atoms; $R^5$ and $R^6$ are, independently, hydrogen or methyl; and $R^7$ and $R^8$ are, independently, alkyl of 1 to 4 carbon atoms, or when taken with the nitrogen atom to which they are attached form a piperidinyl, pyrrolidinyl, morpholinyl, N-alkyl piperazinyl in which the alkyl group contains from

1 to 6 carbon atoms or N-phenylpiperazinyl group; or a pharmaceutically acceptable acid addition salt thereof, which comprises

a) reacting an oxadiazolium salt of the formula:

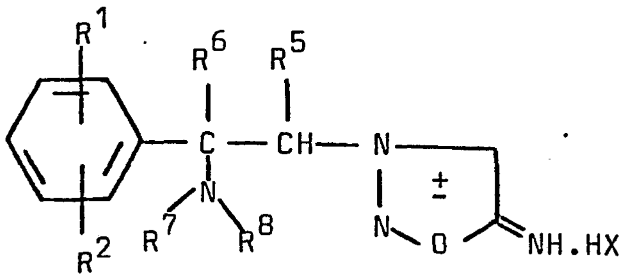

(II)

or the ring opened form of formula:

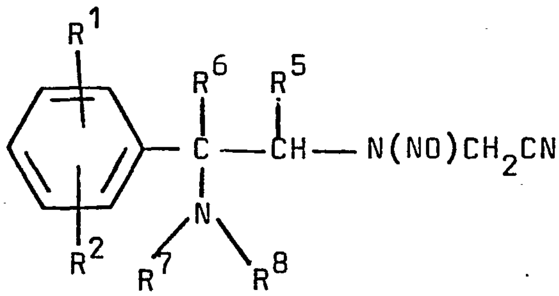

(III)

with an isocyanate of the formula:

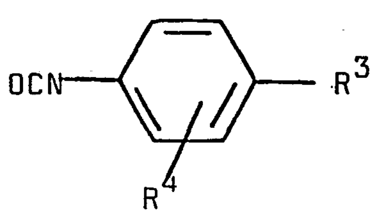

(IV)

in the presence of an organic amine base, in which formula X, $R^1$, $R^2$, $R^3$, $R^4$, $R^5$, $R^6$, $R^7$ and $R^8$ are as defined above to give a compound of formula Ia wherein R is

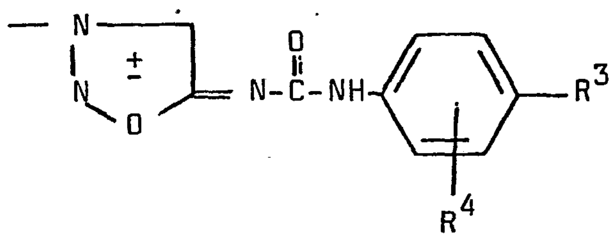

and if desired converting to a pharmaceutically acceptable acid addition salt;

or

b) treating a nitrosonitrile of the formula:

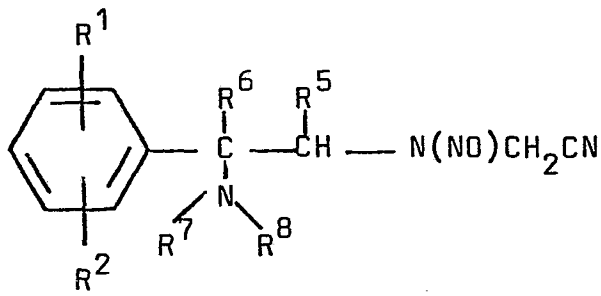

(III)

wherein $R^1$, $R^2$, $R^5$, $R^6$, $R^7$ and $R^8$ are as defined above with excess strong acid to give a compound of formula Ia wherein R is

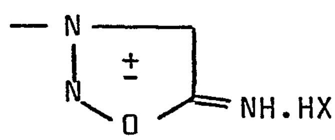

or

c) nitrosating a compound of the formula:

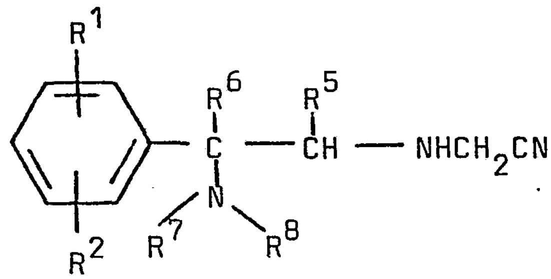

(V)

wherein $R^1$, $R^2$, $R^5$, $R^6$, $R^7$ and $R^8$ are defined above to give a compound of formula Ia wherein R is $-N(NO)CH_2CN$ and if desired converting to a pharmaceutically acceptable salt.

2. A process (a) as claimed in Claim 1 wherein $R^3$ is hydrogen or halogen.

3. A process as claimed in Claim 1 or Claim 2 wherein $R^7$ and/or $R^8$ is methyl.

4. A process as claimed in Claim 1 in which is prepared d-, l- or dl-3-(2-dimethylamino-2-phenylethyl)-N-[(phenylamino)carbonyl]sydnone imine or a non-toxic acid addition salt thereof or d-, l- or dl-3-(2-dimethylamino-2-phenylethyl)-N-[(4-chlorophenylamino)carbonyl]sydnone imine or a non-toxic acid addition salt thereof.

5. A process as claimed in Claim 1 in which is prepared 5-amino-3-[2-(dimethylamino)-2-phenylethyl]-1,2,3-oxadiazolium chloride or a pharmaceutically acceptable acid addition salt thereof, or dl-[(2-dimethylamino)-2-phenylethyl]nitrosoamino]acetonitrile or a pharmaceutically acceptable acid addition salt thereof.

6. A process for preparing a pharmaceutical composition suitable for use as a central nervous system stimulant or as an antihypertensive which comprises mixing together a compound of formula Ia as defined in Claim 1 or a pharmaceutically acceptable acid addition salt thereof and a pharmaceutically acceptable carrier, if desired formulating the composition into unit dosage form.